# EUROPEAN PATENT SPECIFICATION

(11) **EP 1 470 282 B1**
(45) Date of publication and mention of the grant of the patent: **22.02.2017**
(21) Application number: 03705829.4
(22) Date of filing: 17.01.2003
(51) Int. Cl.: D06M 10/00, D06M 10/02, D06M 15/356, D06M 15/61, D06M 15/267, B05D 3/06, A61F 13/511, B05D 3/14, B05D 5/04, B32B 27/16, D06M 11/44, D06M 11/79, D06M 23/08, A61F 13/51, D06M 101/20, D06M 101/32, D06M 11/46

(54) **METHOD FOR HYDROPHILIZING MATERIALS USING HYDROPHILIC POLYMERIC MATERIALS WITH DISCRETE CHARGES**
VERFAHREN ZUR HYDROPHILIERUNG VON MATERIALIEN UNTER VERWENDUNG HYDROPHILER POLYMERER MATERIALIEN MIT DISKRETEN LADUNGEN
PROCEDE POUR RENDRE HYDROPHILE DES MATIERES A L'AIDE DE MATIERES POLYMERIQUES HYDROPHILES PRESENTANT DES CHARGES DISCRETES

(30) Priority: 30.01.2002 US 353049 P
(43) Date of publication of application: 27.10.2004
(73) Proprietor: THE PROCTER & GAMBLE COMPANY, Cincinnati, OH 45202 (US)
(72) Inventor: CRAMER, Ronald, Dean, Cincinnati, OH 45215 (US); ROHRBAUGH, Robert, Henry, Hamilton, OH 45011 (US); CARTER, John, David, Mason, OH 45040 (US); THUEMMIER, Karl, Edward, West Chester, OH 45069 (US)
(74) Representative: Heide, Ute
(86) International application number: PCT/US2003/001643
(87) International publication number: WO 2003/064754

(56) References cited:
- DE-A- 10 029 028
- JP-A- 2001 089 967
- US-A- 5 783 502
- US-A- 5 807 636
- US-A- 5 922 161
- US-A- 5 945 175

## Description

### FIELD OF THE INVENTION

The present invention relates to a method of hydrophilizing or increasing the hydrophilicity of materials having hard and soft surfaces, and more particularly hydrophilizing or increasing the hydrophilicity of such materials by applying a high energy treatment and charged particles and one or more hydrophilic polymeric materials with discrete charges to such hard or soft surface materials.

### BACKGROUND OF THE INVENTION

Hard surface materials include, but are not limited to: metals, glass, wood, stone, fiberglass, plastics, and dishware.

Soft surface materials may include, but are not limited to fabrics, garments, textiles, and films. In certain embodiments, the soft surface materials may comprise one or more structural components, which may include, but are not limited to fibers, yarns, or other types of structural components. The fibers can be formed into numerous structures, including but not limited to nonwoven fabrics and woven or knitted textile fabrics.

Nonwoven materials are widely used in many types of products, including but not limited to disposable absorbent articles, such as diapers, adult incontinence products, and feminine hygiene products.

Many nonwoven materials that are made of synthetic fibers are hydrophobic. It is often desirable to modify such nonwoven materials to make them hydrophilic. Methods for attempting to hydrophilize such nonwoven materials include the use of surfactants. High energy surface treatments have also been used to attempt to hydrophilize nonwoven materials.

A common limitation associated with surfactants is that they tend to wash off the treated material when the treated material is contacted with liquids. This may reduce the effectiveness of nonwoven materials treated with surfactants when the same are used in articles such as disposable absorbent articles that are subject to multiple discharges of liquids such as bodily fluids. A common limitation associated with most high energy surface treatments is durability, particularly on thermoplastic surfaces. The partial or full charges imparted on a thermoplastic surface by various high energy surface treatments tend to dissipate. The technical limitations associated with high energy surface treatments on materials comprised of fibers typically exceed the technical limitations for films of the same material, particularly but not limited to non-perforated films.

Background patent publications include: U.S. Patent 5,618,622; U.S. Patent 5,807,636; U.S. Patent 5,814,567; U.S. Patent 5,922,161; U.S. Patent 5,945,175; U.S. Patent 6,060,410; U.S. Patent 6,217,687; EPO Patent Publication 12513 A1; Japanese Patent Publications JP 55133959 A2; JP 57149363 A2; JP 01141736 A2; JP 05163655 A2; JP 07040514 A2; JP 07233269; JP 9272258; JP 10029660 A2; JP 2000239963 A2; JP 2001270023 A2; and PCT Publications WO 93/12931 A1; WO 97/02310; and WO 01/29118 A1.

One of the foregoing background patent publications, U.S. Patent 5,945,175, is directed to a durable hydrophilic coating for a porous hydrophobic polymer substrate. This publication describes substantially uniformly coating a hydrophobic polymeric material comprised of a hydrophobic polymer with a hydrophilic polymeric material. The hydrophilic polymeric material with which the hydrophobic polymer substrate is coated may be a solution comprising a polysaccharide or a modified polysaccharide. At least a portion of the porous substrate is exposed to a "field of reactive species", and then treated with the hydrophilic polymeric material. Polysaccharide dispersions and solutions are typically viscous and sticky materials, which are often gels that dry very slowly. This publication discloses dipping and immersing corona treated fabrics in aqueous solutions containing the hydrophilic polymeric material, and either drying the fabric in an oven for about 30 minutes, or by using some other process.

JP-A-11181339 discloses a room-temperature-settable coating composition comprising an aqueous fluid containing photo-catalytic titanium oxide particles having a particle diameter of 1-100 nm and tin oxide particles having a particle diameter of 1-100 nm and having a pH of 8-12 or a pH of 0-5. The coating film is said to exhibit hydrophilicity when it is formed on a substrate and irradiated with ultraviolet rays at a wavelength of 200-400 nm and, and the photocatalytic titanium oxide is photo-excited.

A process that applies a viscous and sticky material to a nonwoven material, and requires that the nonwoven material be dried in an oven for 30 minutes would not be suitable for use on a high speed manufacturing line of the type used to make nonwovens or disposable absorbent articles, such as diapers, adult incontinence products, and feminine hygiene products.

Thus, there is a need to provide methods for hydrophilizing or increasing the hydrophilicity of materials, including but not limited to polyolefin nonwoven materials.

### SUMMARY OF THE INVENTION

The present invention relates to a method of hydrophilizing or increasing the hydrophilicity of materials having hard and soft surfaces, as defined in claim 1, and more particularly hydrophilizing or increasing the hydrophilicity of such materials by applying a high energy treatment and charged particles and one or more hydrophilic polymers with discrete charges to such soft surface materials. The hydrophilic polymers with discrete charges may also be referred to herein as "hydrophilic polymeric materials with discrete charges". The charged particles and hydrophilic polymers with discrete charges may also be referred to herein as "charged material" or "charged species".

In one non-limiting embodiment, the method as more specifically defined in claim 1 comprises the steps of:
(a) providing a material comprised of at least some hydrophobic or borderline hydrophilic components;
(b) applying a high energy surface treatment to the material to form a treated material; and
(c) applying a plurality of charged particles and one or more hydrophilic polymers with discrete charges to the treated material.

The high energy surface treatment applied in step (b) can comprise any suitable treatment, including but not limited to: corona discharge treatment, plasma treatment, UV radiation, ion beam treatment, and electron beam treatment. In some embodiments, the charged particles and/or hydrophilic polymers may be applied sequentially, with either treatment applied first, followed by the other treatment. In other embodiments, the charged particles and/or hydrophilic polymers with discrete charges can be applied at the same time as the high energy surface treatment.

In various embodiments, the method described herein can be performed at a number of different stages of processes of preparing the materials that are treated. For example, the method can be perfomed at the following stages: on the structural components (such as fibers, etc.) before they are formed into a structure such as a nonwoven fabric, woven or knitted textile fabrics; on the completed structure (e.g., hard surface, a film, a nonwoven fabric, woven or knitted textile fabrics, etc.); during a process of incorporating the structure into a product (such as a manufacturing line of the type used to make disposable absorbent articles, such as diapers, adult incontinence products, and feminine hygiene products); or, on an article containing the structure (such as a diaper, etc.).

The charged particles and one or more hydrophilic polymers with discrete charges need not be viscous and/or sticky. In some non-limiting embodiments, such as those suited for use on a high speed manufacturing line of the type used to make disposable absorbent articles, such as diapers, adult incontinence products, and feminine hygiene products, the method may be carried out in less than 30 minutes. In some embodiments, the method can be carried out in a matter of seconds.

### BRIEF DESCRIPTION OF THE DRAWINGS

While the specification concludes with claims particularly pointing out and distinctly claiming the invention, it is believed that the present invention will be better understood from the following description taken in conjunction with the accompanying drawings in which:

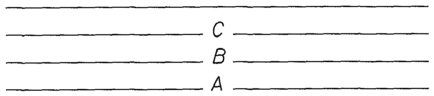

Fig. 1 is a schematic side view which is used to illustrate various embodiments of a substrate that is treated according to the method described herein.

### DETAILED DESCRIPTION OF THE INVENTION

The present invention relates to a method of hydrophilizing materials or increasing the hydrophilicity of materials. The materials comprise soft surface materials.

Soft surface materials may include, but are not limited to fabrics, garments, textiles, and films. In certain embodiments, the soft surface materials may comprise one or more structural components, which may include, but are not limited to fibers, yarns, or other types of structural components. The fibers can be formed into numerous structures, including but not limited to nonwoven fabrics and woven or knitted textile fabrics.

The fibers can be comprised of natural materials, man-made materials, or combinations thereof. Natural fibers include, but are not limited to: animal fibers such as wool, silk, fur, and hair; vegetable fibers such as cellulose, cotton, flax, linen, and hemp; and certain naturally occurring mineral fibers. Synthetic fibers can be derived from natural fibers. Example synthetic fibers which are derived from natural fibers include but are not limited to rayon and lyocell. Synthetic fibers can also be derived from other natural sources or from mineral sources. Example synthetic fibers derived from natural sources other than natural fibers include but are not limited to certain polysaccharides such as starch. Example fibers from mineral sources include but are not limited to polyolefin fibers such as polypropylene and polyethylene fibers. Some synthetic fibers can be comprised of materials that are thermoplastic or thermoset materials. Synthetic fiber resins can be homo-polymers, co-polymers, polymer blends, or combinations thereof. Common synthetic fiber resins include but are not limited to nylon (polyamide), acrylic (polyacrylonitrile), aramid (aromatic polyamide), polyolefin (polyethylene and polypropylene), polyester, butadiene-stryene block copolymers, natural rubber, latex, and spandex (polyurethane). The fibers can also be multicomponent fibers, including but not limited to bicomponent fibers.

Nonwoven materials are a type of fabric typically made from fibers in a web format. Nonwoven webs are described by Butler I, Batra SK, et al, Nonwovens Fabrics Handbook, Association of the Nonwoven Fabrics Industry, 1999, and by Vaughn EA, Nonwoven Fabric Sampler and Technology Reference, Association of the Nonwoven Fabrics Industry.

Nonwoven webs can be formed by direct extrusion processes during which the fibers and webs are formed at about the same point in time, or by preformed fiber processes (laying processes) in which fibers can be laid into webs at a distinctly subsequent point in time following fiber formation. Example direct extrusion processes include but are not limited to: spunbonding, meltblowing, solvent spinning, electrospinning, and combinations thereof typically forming layers. Example laying processes include wetlaying and drylaying. Example drylaying processes include but are not limited to airlaying, carding, and combinations thereof typically forming layers. Combinations of the above processes yield nonwovens commonly called hybrids or composites. Example combinations include but are not limited to spunbond-meltblown-spunbond (SMS), spunbond-carded (SC), spunbond-airlaid (SA), meltblown-airlaid (MA), and combinations thereof, typically in layers. Combinations which include direct extrusion can be combined at the about the same point in time as the direct extrusion process (e.g., spinform and coform for SA and MA), or at a subsequent point in time. In the above examples, one or more individual layers can be created by each process. For instance, SMS can mean a three layer, "sms" web, a five layer "ssmms" web, or any reasonable variation thereof wherein the lower case letters designate individual layers and the upper case letters designate the compilation of similar, adjacent layers.

Most fibers in most nonwoven webs are typically oriented with some degree of relative angle to at least a portion of one or more other fibers. Places where two or more fibers touch are called junctions. Junctions can be adjacent or overlapping with some degree of relative angle therebetween. The fibers in a nonwoven web are typically joined to one or more adjacent fibers at some of the junctions. This includes joining fibers within each layer and joining fibers between layers when there is more than one layer. Common approaches to joining fibers include but are not limited to mechanical entanglement, chemical bonding, or combinations thereof. Example fiber joining processes include but are not limited to thermal bonding, pressure bonding, ultrasonic bonding, solvent bonding, stitchbonding, needlepunching, and hydroentanglement. The joining processes can optionally include an intermediary material. Example optional intermediary materials include but are not limited to binders such as a binding fibers, solvents, and threads.

Fibers and nonwoven webs can be subjected to additional treatment after formation. For nonwoven webs, additional treatment commonly occurs after the fibers are joined to one another (post-treatment). Examples of additional treatments include but are not limited to mechanical stresses, chemical additives, or combinations thereof. Chemical additive approaches are well known in the art. Chemical additives can be applied around a portion of or around entire individual fibers, to one side of a web, or to both sides of a web by a variety of techniques many of which can apply chemical additives to a portion of the fibers or web, or to all fibers or to the entire web over various timeframes. Chemicals can be added from a solid phase, a liquid phase, a gaseous phase, or as the result of a high energy surface treatment including but not limited to irradiation, irradiative oxidation, or plasma treatment. High energy surface treatments can also be used to promote chemical changes of the material(s) on or near the fiber surface. Example high energy surface treatments include but are not limited to corona discharge treatment, plasma treatment, UV radiation treatment, ion beam treatment, electron beam treatment, and certain laser treatments including pulsed lasers. Additives or chemical changes on or near the fiber surface resulting from certain high energy surface treatments include but are not limited to the creation of ozone from atmospheric oxygen near the surface, the establishment of free radicals or electrons or other partial or fully charged species on the surface, and the crosslinking of candidate macromolecules in the surface.

The limitations associated with high energy surface treatments of materials comprised of fibers typically exceed the limitations for films of the same material, particularly but not limited to non-perforated films. Without wishing to be bound by any particular theory, a key distinction is the surface geometry. While films have a three dimensional surface topography at the nanoscopic level, films can be regarded, for the purposes of high energy surface treatment in comparison to fibers, as being approximately two dimensional, or planar, at higher scales (length and width dominate thickness which only becomes relevant at edges). The three dimensional geometry of fibers, including fibrous fabrics, makes the thickness dimension more relevant than for films. In comparison to many films, the plurality of fibers creates a plurality of cross-planar, or z-direction, edges which constitute surface area. Furthermore, most fabrics have fiber surfaces which are not adjacent to an imaginary macroscopic plane which can be drawn across a plurality of the outermost fiber edges on either side of a fabric. Indeed, portions of the non-adjacent fiber surfaces can often be regarded as hidden zones. Applying high energy surface treatments or any resultant species created by a high energy surface treatment to partially or fully penetrate hidden zones in a reasonable timeframe is a limitation associated with most fibrous fabrics. This type of limitation is sometimes called shadowing. In contrast, common films such as a non-perforated film comprised of the same material as a fibrous fabric, with surface area and nanoscale topography comparable to the fibrous surface area, has fewer hidden zones. When exposed to a comparable dose from a high energy surface treatment, a greater portion of the surface area of said film is thus exposed in comparison to said fibrous fabric. This typically yields a higher charge density on average for a film surface than for the surfaces of the fibers in a fabric. As the charge dissipates, the fibrous fabric limitations continue. The fibrous fabric has a greater surface area across which to dissipate the charge which is initially primarily located on the fiber surfaces facing outward.

Nonwoven webs are commonly joined with other nonwoven webs or films forming composite nonwoven webs. Such webs can be joined in ways previously described and are commonly called nonwoven laminates. A non-limiting example nonwoven laminate is a disposable absorbent product backsheet such as a diaper backsheet in which a nonwoven is joined to a film such as a microporous film. Variations of the length, width, materials, etc. of various layers in a nonwoven laminate yield complex nonwoven webs. A disposable absorbent product web prior to being cut into individual segments, typically into finished product segments, is an example of a nonwoven laminate web and, typically, of a complex nonwoven web. For the purposes of this invention, all webs which comprise a nonwoven are considered a nonwoven. This includes but is not limited to nonwoven webs, composite nonwoven webs, nonwoven laminates, and complex nonwoven webs.

Hydrophobic or borderline hydrophilic soft surfaces include, but are not limited to textile materials such as knitted, woven, and nonwoven materials that are comprised of hydrophobic or borderline hydrophilic structural components. The structural components of a knitted, woven, or nonwoven material may comprise yarns, strands, fibers, threads, or other structural components. Some or all of the structural are components are hydrophobic, borderline hydrophilic, or combinations thereof. Hydrophobic structural components are those that entirely comprise a hydrophobic material, or partially comprise a hydrophobic material on the surface (such as a multi-component fiber comprising a core of one or more materials partially or fully surrounded by a hydrophobic sheath). Similarly, borderline hydrophilic structural components are those that entirely comprise a borderline hydrophilic material or partially comprise a borderline hydrophilic material on the surface. If a structural component includes both hydrophobic materials and borderline hydrophilic materials on the surface, then it is considered hydrophobic. Hydrophobic materials are often synthetic homo-polymers, co-polymers, polymer blends, or combinations thereof. Examples include but are not limited to polyolefins such as polypropylene and polyethylene, certain polyesters such as polyethylene terepthalate (PET), and certain polyamides. Borderline hydrophilic materials are also often synthetic homo-polymers, co-polymers, polymer blends, or combinations thereof. Examples include but are not limited to certain polyesters which exhibit borderline hydrophilicity. Polyesters which exhibit borderline hydrophilicity include the class of polyesters which have recently been termed hydrophilic polyesters. One example is PET/branched polyethylene glycol (branched PEG) copolymers such as the T870, T289, and T801 grades available from Wellman, Inc., Charlotte, NC, USA. Another example is polyesters with aliphatic repeat units instead of some or all of the aromatic repeat units of PET. Polylactide (or polylactic acid or PLA) polymers available from Cargill Dow Polymers, LLC, Blair Nebraska contain aliphatic repeat units. Eastar Bio^{®} brand biodegradable copolyester, a poly(tetramethylene adipate-co-terepthalate), or PTAT, available from Eastman Chemical Company, Kingsport Tennessee, is a similar example.

While surfactants may work well for hydrophilizing or increasing the hydrophilicity of fibers for many applications, in the case of some of the hydrophobic or borderline hydrophilic materials described above, use of surfactant may be particularly problematic when the material is rewetted during use, such as in articles which transport fluid including but not limited to textiles, absorbent articles and disposable absorbent articles such as diapers and other incontinence and catamenial products such as feminine pads, that are subject to one or more gushes of liquid during use (e.g., urine, menses, sweat, or other body exudates). Liquid gushes wash surfactant from the soft surface into the liquid phase itself during use. Even low levels of surfactant in the liquid phase reduces the surface tension of the liquid. Reduced surface tension in the liquid phase lowers the liquid wicking tension along the fibers (where wicking tension equals surface tension multiplied by the cosine of the contact angle). Lower wicking tension reduces the wicking velocity and, in turn, the wicking flux through or along the porous fabric (amount of liquid per unit time per unit cross sectional area). Reduced wicking flux can result in lower liquid handling performance to the end user.

Reduced surface tension in the liquid phase also increases its ability to wet fabric surfaces which are intentionally hydrophobic. Once a formerly hydrophobic fabric is wetted, it can begin exhibiting hydrophilic behavior. A hydrophobic surface which otherwise would have repelled a fluid such as water can pass the fluid through or along the fabric via wicking tension force, gravitational force, pressure gradient force, or other forces. One example is an SMS barrier leg cuff of a diaper through which pure urine cannot easily pass under most use conditions. The reduced surface tension of urine contaminated with surfactant can enable wetting and subsequent passage through said SMS fabric. This can result in the perception of leakage by the end user.

An alternative to reducing fluid surface tension for the purposes of improving the extent to which a liquid will wet a soft surface is to more durably increase surface energy of the material. It has been found that materials that have been subjected to a high energy surface treatment and have a plurality of charged particles and/or one or more hydrophilic polymers with discrete charges applied thereto will have a more durable increase in surface energy. In some embodiments, such a method will result in treated materials that will have a minimal reduction in surface tension, and are not surface active, or are minimally surface active.

High energy surface treatments can include, but are not limited to: corona discharge treatment, plasma treatment, UV radiation treatment, ion beam treatment, electron beam treatment, certain laser treatments including pulsed lasers, and other irradiative techniques, provided the surface energy of a portion of some of the fibers is increased. In some embodiments, it may be desirable for care to be taken to avoid adversely affecting the material to be treated.

### Charged Particles

The charged particles used herein can be either positively charged, or negatively charged, or they can contain both positive and negative charges. It should be understood that every limit given throughout this specification will include every lower, or higher limit, as the case may be, as if such lower or higher limit was expressly written herein. Every range given throughout this specification will include every narrower range that falls within such broader range, as if such narrower ranges were all expressly written herein.

Nanoparticles may be advantageous if it is desirable for the particles to be invisible on the material to which the charged particles are applied. In certain embodiments, such as when the material to which the charged particles are applied is enclosed in the interior of an absorbent article, it may not be important that some of the charged particles would otherwise be visible if the treated material was exposed. In some embodiments, where the particles are applied to fibrous materials, it may be desirable for the particles to beless than or equal to the width (e.g., diameter) of the fibers to which they are applied.

The particles are less than or equal to 10 microns in size, or any number of microns less than 10 microns in size, including equal to 5 microns. The charged particles can all be within a certain range of sizes, or they can comprise a range of particle sizes that are mixed together.

The charged particles can comprise any suitable material or materials. The charged particles can be comprised of natural and synthetic materials. The charged particles can be organic, or inorganic. The charged particles may be insoluble in water and other mediums. The charged particles may be photoactive or non-photoactive. Photoactive particles are particles that require UV or visible light to activate the particles whereby the particles become more hydrophilic.

Suitable materials from which the charged particles can be selected include but are not limited to the following materials: organic particles such as latexes; inorganic particles such as oxides, silicates, carbonates and hydroxides, including some layered clay minerals and inorganic metal oxides.

The layered clay minerals suitable for use herein include those in the geological classes of the smectites, the kaolins, the illites, the chlorites, the attapulgites and the mixed layer clays. Smectites include montmorillonite, bentonite, pyrophyllite, hectorite, saponite, sauconite, nontronite, talc, beidellite, volchonskoite and vermiculite. Kaolins include kaolinite, dickite, nacrite, antigorite, anauxite, halloysite, indellite and chrysotile. Illites include bravaisite, muscovite, paragonite, phlogopite and biotite. Chlorites include corrensite, penninite, donbassite, sudoite, pennine and clinochlore. Attapulgites include sepiolite and polygorskyte. Mixed layer clays include allevardite and vermiculitebiotite. Variants and isomorphic substitutions of these layered clay minerals offer unique applications.

Layered clay minerals may be either naturally occurring or synthetic. Layered clay minerals include natural or synthetic hectorites, montmorillonites and bentonites. Typical sources of commercial hectorites are the LAPONITEs™ from Southern Clay Products, Inc., U.S.A; Veegum Pro and Veegum F from R. T. Vanderbilt, U.S.A.; and the Barasyms, Macaloids and Propaloids from Baroid Division, National Read Comp., U.S.A.

Natural clay minerals typically exist as layered silicate minerals and less frequently as amorphous minerals. A layered silicate mineral has SiO₄ tetrahedral sheets arranged into a two-dimensional network structure. A 2:1 type layered silicate mineral has a laminated structure of several to several tens of silicate sheets having a three layered structure in which a magnesium octahedral sheet or an aluminum octahedral sheet is sandwiched between two sheets of silica tetrahedral sheets.

A sheet of an expandable layer silicate has a negative electric charge, and the electric charge is neutralized by the existence of alkali metal cations and/or alkaline earth metal cations. Smectite or expandable mica can be dispersed in water to form a sol with thixotropic properties. Further, a complex variant of the smectite type clay can be formed by the reaction with various cationic organic or inorganic compounds. As an example of such an organic complex, an organophilic clay in which a dimethyldioctadecyl ammonium ion (a quaternary ammonium ion) is introduced by cation exchange and has been industrially produced and used as a gellant of a coating.

The production of nanoscale powders such as layered hydrous silicate, layered hydrous aluminum silicate, fluorosilicate, mica-montmorillonite, hydrotalcite, lithium magnesium silicate and lithium magnesium fluorosilicate are common. An example of a substituted variant of lithium magnesium silicate is where the hydroxyl group is partially substituted with fluorine. Lithium and magnesium may also be partially substituted by aluminum. In fact, the lithium magnesium silicate may be isomorphically substituted by any member selected from the group consisting of magnesium, aluminum, lithium, iron, chromium, zinc and mixtures thereof.

LAPONITE™, a lithium magnesium silicate has the formula:

[Mg_{w}LiₓSi₈O₂₀OH_{4-y}F_{y}]^{z-}

wherein w = 3 to 6, x = 0 to 3, y = 0 to 4, z = 12 - 2w - x, and the overall negative lattice charge is balanced by counter-ions; and wherein the counter-ions are selected from the group consisting of selected Na⁺, K⁺, NH₄⁺, Cs⁺, Li⁺, Mg⁺⁺, Ca⁺⁺, Ba⁺⁺, N(CH₃)₄⁺ and mixtures thereof. (If the LAPONITE™ is "modified" with a cationic organic compound, then the "counter-ion" could be viewed as being any cationic organic group (R).)

There are many grades or variants and isomorphous substitutions of LAPONITE™ marketed. Examples of commercial hectorites are LAPONITE B™, LAPONITE S™, LAPONITE XLS™, LAPONITE RD™, LAPONITE XLG™, and LAPONITE RDS™. LAPONITE XLS™ has the following characteristics: analysis (dry basis) SiO₂ 59.8%, MgO 27.2%, Na₂ O 4.4%, Li₂ O 0.8%, structural H₂O 7.8%, with the addition of tetrasodium pyrophosphate (6%); specific gravity 2.53; bulk density 1.0.

Some synthetic hectorites, such as LAPONITE RD™, do not contain any fluorine. An isomorphous substitution of the hydroxyl group with fluorine will produce synthetic clays referred to as sodium magnesium lithium fluorosilicates. These sodium magnesium lithium fluorosilicates, marketed as LAPONITE™ and LAPONITE S™, may contain fluoride ions of up to approximately 10% by weight. LAPONITE S™, contains about 6% of tetrasodium pyrophosphate as an additive.

Depending upon the application, the use of variants and isomorphous substitutions of LAPONITE™ provides great flexibility in engineering the desired properties of compositions used in carrying out the present invention. The individual platelets of LAPONITE™ are negatively charged on their faces and possess a high concentration of surface bound water. When delivered from a water or water/surfactant or water/alcohol/surfactant carrier medium, the surface may be hydrophilically modified. Such surfaces may, depending on the embodiment, (e.g., in the case of soft surfaces) exhibit surprising and significantly improved wettability, strike-through, comfort.

Inorganic metal oxides generally fall within two groups--photoactive and non-photoactive particles. General examples of photoactive metal oxide particles include zinc oxide and titanium oxide. Photoactive metal oxide particles require photoactivation from either visible light (e.g. zinc oxide) or from UV light (TiO₂).

The inorganic metal oxides may be silica- or alumina- based particles that are naturally occurring or synthetic. Aluminum can be found in many naturally occurring sources, such as kaolinite and bauxite. The naturally occurring sources of alumina are processed by the Hall process or the Bayer process to yield the desired alumina type required. Various forms of alumina are commercially available in the form of Gibbsite, Diaspore, and Boehmite from manufacturers such as Condea, Inc.

Non-photoactive metal oxide particles do not use UV or visible light to produce the desired effects. Examples of non-photoactive metal oxide particles include, but are not limited to: silica, zirconium oxide, aluminum oxide, magnesium oxide, and boehmite alumina nanoparticles, and mixed metal oxide particles including, but not limited to smectites, saponites, and hydrotalcite.

Boehmite alumina ([Al(O)(OH)]ₙ) is a water dispersible, inorganic metal oxide that can be prepared to have a variety of particle sizes or range of particle sizes, including a mean particle size distribution from about 2 nm to less than or equal to about 750 nm. A boehmite alumina nanoparticle with a mean particle size distribution of around 25 nm under the trade name Disperal P2™ and a nanoparticle with a mean particle size distribution of around 140 nm under the trade name of Dispal® 14N4-25 are available from North American Sasol, Inc.

A "latex" is a colloidal dispersion of water-insoluble polymer particles that are usually spherical in shape. A "nanolatex", as used herein, is a latex with particle sizes less than or equal to about 750 nm. Nanolatexes may be formed by emulsion polymerization. "Emulsion polymerization" is a process in which monomers of the latex are dispersed in water using a surfactant to form a stable emulsion followed by polymerization. Particles are produced with can range in size from about 2 to about 600 nm.

### The Hydrophilic Polymeric Material With Discrete Charges

The method uses hydrophilic polymers (or hydrophilic polymeric material) in addition to, charged particles. The hydrophilic polymers: should have discrete charges (or one or more charged groups) associated therewith; comprise hydrophilic polymers with a strong dipole; or comprise hydrophilic polymers with both discrete charges and a strong dipole moment. The hydrophilic polymers may also comprise soil release polymers comprising discrete charges, especially those with sulfonate groups. It should be understood that if the phrase "hydrophilic polymers with discrete charges" is used herein in reference to the method described herein, any such references will also apply to the other groups of polymers referred to above, such as polymers with a strong dipole and hydrophilic polymers other than polysaccharides.

The hydrophilic polymers can be synthetic (as opposed to polysaccharides, which are typically natural or derivatives of natural polysaccharide materials, such as sugars and starches). The hydrophilic polymers can be non-polysaccharides. The present invention, however, can utilize a first group of hydrophilic polymers as described above, and does not exclude the use of some hydrophilic polymers of other types, including but not limited to polysaccharides in a second or additional group of hydrophilic polymers.

The hydrophilic polymers with discrete charges can be cationic, anionic, or zwitterionic. When it is said that the hydrophilic polymers have a strong dipole, this refers to the dipole moment of their functional group, rather than the dipoles of the entire polymer. The hydrophilic polymers may have any suitable molecular weight. In some embodiments, it is desirable for the hydrophilic polymers to have a lower molecular weight than polysaccharides and polysaccharide derivatives for ease of application, and to reduce drying time. In some embodiments, it may be desirable for the hydrophilic polymers to have molecular weights of less than or equal to about 500,000 Daltons, or any number or range of numbers less than 500,000 (including, but not limited to 200,000 to 300,000 Daltons).

The hydrophilic polymers maybe homopolymers, random copolymers, block copolymers or graft copolymers. The hydrophilic polymers may be linear, branched or dendritic.

### Polycationics

By way of illustration, polycationic species may contain two or more quaternary ammonium groups with a molecular weight ranging from several hundred Daltons to a few hundred thousand Daltons. The quaternary ammonium groups may be part of a ring or they may be acyclic. Examples include but are not limited to: polyionenes, poly(diallyldimethylammonium chloride), dimethylamine-epichlorohydrin copolymers and imidazole-epichlorohydrin copolymers.

In a further illustration, the polycationic species may contain two or more amine groups. The amine groups can be primary, secondary, tertiary, or mixtures thereof. The amine groups may be part of a ring or they may be acyclic. Examples include but are not limited to: polyethyleneimines, polypropyleneimines, polyvinylamines, polyallylamines, polydiallylamines, polyamidoamines, polyaminoalkylmethacrylates, polylysines, and mixtures thereof.

The polycationic species may also be a modified polyamine with at least one amine group substituted with at least one other functional group. Examples include ethoxylated and alkoxylated polyamines and alkylated polyamines.

### Zwitterionics

The zwitterionic species may contain two or more amine groups with at least one amine group quaternized and at least one amine group substituted by one or more moieties capable of bearing an anionic charge.

In a further illustration, the zwitterionic species may contain two or more amine groups with at least one amine group substituted by one or more moieties capable of bearing an anionic charge. Examples include: polyamine oxides, oxidized ethoxylated polyethyleneimine, carboxymethylated polyethyleneimine, maleated polyethyleneimine and ethoxylated, sulfated polyethyleneimine.

### Polyanionics

The polyanionic species may contain water soluble anionic groups including but not limited to: carboxylates, sulfonates, sulfates, phosphates, phosphonates and mixtures thereof. Examples include but are not limited to: polyacrylates, polymethacrylates, polymaleates, polyitaconates, polyaspartates, polyglyoxylates, polyvinylsulfates, polyvinylsulfonates, polystyrenesulfonates, aldehyde condensates of naphthalene napthalenesulfonic or phenolsulfonic acid, copolyesters comprising sulfoisophthalate, copolyesters comprising teraphthalates and sulfonated allylethoxylates groups, copolyesters comprising diolsulfonates, poly(2-acrylamido-2-methylpropanesulfonic acid) and copolymers thereof.

### Hydrophilic Polymeric Materials With a Strong Dipole

Hydrophilic polymeric materials with a strong dipole can comprise monomer groups with high dipole moments such as amide groups. Examples include but are not limited to: polyvinylpyrrolidones, polyacrylamides, polyvinyloxazolines, and copolymers thereof.

### Other Charged Materials

In addition to charged particles and hydrophilic polymeric materials with discrete charges, multi-valent inorganic salts may be used in certain embodiments of the method. The multi-valent inorganic salts may serve to anchor or enhance adsorption of the charged particles and/or polymeric materials with discrete charges onto the surfaces. Multi-valent inorganic salts can be selected from the group consisting of Ca⁺², Mg⁺², Ba⁺², Al⁺³, Fe⁺², Fe⁺³, Cu⁺² and mixtures thereof, where an appropriate anion is used to balance the charge.

Fig. 1 can be used to illustrate several non-limiting embodiments of a substrate that is treated according to the method described herein. In Fig. 1, the substrate is represented by reference letter A. Reference letter B is a "primer" or "basecoat". Reference letter C can be used to refer to a treatment (e.g., an "active" treatment) applied on top of the basecoat. The primer or basecoat may be positively charged, or negatively charged. The treatment "C" may be positively charged or negatively charged. It should be understood that Fig. 1 is only a schematic representation, and the structures formed by the methods described herein are not limited to structures that form layer-type arrangements such as that shown in Fig. 1. For example, in some embodiments, the "layer" may not be visible. In other embodiments, the "layer" will actually be comprised of a plurality of particles distributed on and/or within the surface of a substrate. In still other embodiments, there may be more than the number of "layers" or treatments shown in Fig. 1.

In various embodiments, the high energy treatment can be considered to be the basecoat or primer. Alternatively, the basecoat or primer could be the charged particles or the polymeric material having discrete charges. In these embodiments, the treatment, reference letter C, can comprise the charged particles or the polymeric material having discrete charges.

Thus, the hydrophilic modification of a surface (or substrate) can be augmented via use of particles, including nanoparticles such as LAPONITE™ as a basecoat or primer and then treating the negatively charged surface with a hydrophilic polymer having discrete charges as a two-step process. Additional coatings of the nanoparticles and hydrophilic polymer having discrete charges can be added if desired, for example to provide alternating layers of the same in a process involving more than two steps.

In other embodiments, for example, a substrate that has been subjected to a high energy treatment can be designated by reference letter A. In one version of such an embodiment, the charged particles can serve as primers/basecoats (layer B) on the high energy treated surface. This can be subsequently treated with hydrophilic polymers with discrete charges to form layer C (e.g., alumina followed by polyanionic species). In another version of such an embodiment, the hydrophilic polymers with discrete charges can be used as primers/basecoats (layer B) on the high energy treated surfaces (layer A) which is then subsequently treated with charged particles to form "layer" C (e.g. polydiallyldimethylammonium chloride followed by LAPONITE™). Other embodiments can use a combination of charged particles and other charged hydrophilic species.

Sequential layering of LAPONITE™ and ethoxylated, quaternized oligoamines results in a reduction in the contact angles, and enhanced sheeting/wetting of the treated surface. Thus, the combination of nanoclay plus a hydrophilic polymer having discrete charges may be used to provide a novel technique for tailoring the hydrophilic/lipophilic character of a surface. Similarly, sequential layering of alumina and hydrophilic anionic polymers results in enhanced sheeting/wetting of the treated surface. Thus, the combination of inorganic metal oxides plus hydrophilic polymers with charges may be used to provide a novel technique for tailoring the hydrophilic/lipophilic character of a surface.

In still other embodiments, any of the particles described herein can be modified with the other materials described herein, such as the hydrophilic polymeric material with discrete charges or the other charged materials, before the particles are applied to the surface. These modified particles can then be applied to the surface with or without having applied the high energy treatment to the surface.

Surfactants are an optional ingredient in some embodiments of the compositions used herein. Surfactants may be useful in the composition as wetting agents to facilitate the dispersion of particles and/or polymeric material onto a surface. Surfactants are alternatively included when the composition is used to treat a hydrophobic soft surface or when the composition is applied with in a spray dispenser in order to enhance the spray characteristics of the composition and allow the coating composition, including the particles, to distribute more evenly. The spreading of the coating composition can also allow it to dry faster, so that the treated material is ready to use sooner. When a surfactant is used in the composition, may be added at an effective amount to provide facilitate application of the coating composition. Suitable surfactants can be selected from the group including anionic surfactants, cationic surfactants, nonionic surfactants, amphoteric surfactants, ampholytic surfactants, zwitterionic surfactants and mixtures thereof. Examples of suitable nonionic, anionic, cationic, ampholytic, zwitterionic and semi-polar nonionic surfactants are disclosed in U.S. Pat. Nos. 5,707,950 and 5,576,282.

The charged particles and one or more hydrophilic polymeric materials with discrete charges can be applied to the surface to be treated (or substrate) in any suitable manner including, but not limited to incorporating the charged particles and

one or more hydrophilic polymeric materials with discrete charges in a composition, and applying the composition to the surface to be treated. The composition may be in any form, such as liquids (aqueous or non-aqueous), granules, pastes, powders, spray, foam, tablets, gels, and the like.

The charged particles and the hydrophilic polymeric materials may be incorporated into such a composition in any suitable amount up to 100%. For example in some embodiments, the composition can be sprayed on neat from a 100% solution of the hydrophilic polymeric material.

The composition can be applied to in any suitable quantity to the material to be treated. In some embodiments in which the composition is applied to a material having a soft surface, the composition can be applied in an amount ranging from about 0.05 and about 10% of the weight of the material. The amount of the composition may also fall within any narrower range within such a range, including but not limited to between about 0.1% and about 10%, between about 0.2% and about 5%, and between about 0.2% and about 2%.

The composition can be applied to the material to be treated in any suitable manner, including, but not limited to: by adding the coating composition in a washing and/or rinsing process, by spraying, dipping, painting, wiping, printing, or by any other manner. If the composition is applied to the material by spraying, the viscosity of the composition should be suitable for spraying (e.g., the composition should be a liquid), or if the composition is in some other form, such as a gel, the composition should be capable of shear thinning to form a liquid that is capable of being sprayed. The composition can be applied to the surface of the material, and if the material is porous, and/or to interior portions of the material.

The composition may, but need not, substantially uniformly coat the material to which it is applied. The composition may completely cover a surface, or portion thereof (e.g., continuous coatings, including those that form films on the surface), or it may only partially cover a surface, such as those coatings that after drying leave gaps in coverage on a surface (e.g., discontinuous coatings). The later category may include, but is not limited to a network of covered and uncovered portions and distributions of particles on a surface which may have spaces between the particles. In addition, when the composition or coating described herein is described as being applied to a surface, it is understood that they need not be applied to, or that they cover the entire surface. For instance, the coatings will be considered as being applied to a surface even if they are only applied to modify a portion of the surface.

In various embodiments, the method described herein can be performed at a number of different stages of processes that utilize the materials that are treated. For example, the method can be perfomed at the following stages: on the completed structure (e.g., hard surface, a film, a nonwoven fabric, woven or knitted textile fabrics, etc.); during a process of incorporating the structure into a product (such as a manufacturing line of the type used to make disposable absorbent articles, such as diapers, adult incontinence products, and feminine hygiene products); or, on the structure itself (such as on a nonwoven material), or on an article containing the structure (such as a diaper).

In some non-limiting embodiments, such as those suited for use on a high speed manufacturing line of the type used to make disposable absorbent articles, such as diapers, adult incontinence products, and feminine hygiene products, the method may be carried out in less than 30 minutes, or any number of minutes less than 30 minutes. In some embodiments, the method can be carried out in a matter of seconds, including any number of seconds less than or equal to 60 seconds. To accelerate drying, the substrate may be heated to any temperature below its melting temperature.

In some cases, it may be desirable for some of these treatments to be applied to both sides of a soft surface. In addition, it is contemplated that this optional step may be a separate, pre-treatment step from the application of the charged particles and/or one or more hydrophilic polymeric materials with discrete charges to the material to be treated, or these two steps may be combined.

As discussed earlier, the partial or full charges from a high energy surface treatment dissipate over time, and maintaining partial or full charges on fibrous thermoplastic surfaces is a common limitation. However, in a non-limiting example, it has been found that corona treatment in combination with the charged particles and/or one or more hydrophilic polymeric materials with discrete charges can be used to place a more durable charge on the material so that water based fluids continue to be attracted to the material after time elapses or after multiple fluid insults. The use of charged particles and/or one or more hydrophilic polymeric materials with discrete charges in conjuction with high energy surface treatments, can convert the transient properties of said treatments to more durable properties.

The materials that have been subjected to a high energy surface treatment and have a plurality of charged particles and one or more hydrophilic polymeric materials with discrete charges deposited thereon can be suitable for a great many uses including, but not limited to use to transport liquid in articles such as clothing containing hydrophobic or borderline hydrophilic fibers, in articles used for wiping hard and soft surfaces, and in portions of absorbent articles including disposable absorbent articles. The articles used for wiping hard or soft surfaces may include pre-moistened wipes and dry wipes. Pre-moistened wipes may be saturated with one or more liquids such as a wet wipe or unsaturated with one or more liquids such as a moist wipe. The wipes may be disposable or reusable. Examples of types of wipes include but are not limited to skin wipes such as baby wipes, feminine wipes, anal wipes, and facial wipes; to household cleaning wipes such as floor wipes, furniture wipes, and bathroom wipes; and to automobile wipes. The portions of disposable absorbent articles include but are not limited to topsheets, acquisition layers, distribution layers, wicking layers, storage layers, absorbent cores, absorbent core wraps and containment structures.

In some embodiments, the liquid strike-through time of a material treated in such a manner is less than or equal to about 10 seconds, preferably less than or equal to about 6 seconds, more preferably less than or equal to about 3 seconds, after 3 gushes of test liquid, or any higher number of liquid insults, including but not limited to after 5 gushes of test liquid, and after 10 gushes of test liquid, when tested in accordance with the Strike-Through Test in the Test Methods section.

The materials that have been treated with the coating composition described herein for the purpose of rendering them hydrophilic, regardless of whether they have been subjected to the high energy surface treatment, may be made to have advancing contact angles with water of less than or equal to 90°, or less than 90°, or any number of degrees less than 90, including but not limited to 45°, after 30 seconds of spreading.
Even though none of the following examples falls within the scope of the present invention, they are generally illustrative of the present invention. All parts, percentages and ratios used herein are expressed as percent weight unless otherwise specified.

### EXAMPLES

Strike through results for SMS polypropylene nonwoven materials (13 grams per square meter) exposed to a Laboratory Corona Treater (Mode1# BD-20AC, manufactured by Electro-Technic Products Inc., USA) and coating compositions are reported in the following Table (wherein the balance of the composition comprises water).

| Composition Applied to Nonwoven | Corona Treatment | Strike Through Times / seconds | | |
|---|---|---|---|---|
| | | 1^{st} Insult | 2^{nd} Insult | 3^{rd} Insult |
| None | No | >120 | - | - |
| None | Yes | 10-18 | 6-10 | 4-10 |
| 0.2% Laponite RD¹ | No | >120 | - | - |
| 0.2% Laponite RD¹ | Yes | 4.7 | 3.2 | 2.8 |
| 0.2% Disperal P2² | No | >120 | - | - |
| 0.2% Disperal P2² | Yes | 2.1 | 2.3 | 2.3 |
| 0.2% Polyethyleneimine, MW=3000 | No | >120 | - | - |
| 0.2% Polyethyleneimine, MW=3000 | Yes | 1.3 | 1.6 | 1.8 |
| 0.2% Polydiallydimethylammonium chloride³, very low MW | No | >120 | - | - |
| 0.2% Polydiallydimethylammonium chloride³, very low MW | Yes | 4.7 | 2.5 | 2.4 |
| 0.2% Polyacrylic acid, sodium salt⁴ MW=3500 | No | >120 | - | - |
| 0.2% Polyacrylic acid, sodium salt⁴ MW=3500 | Yes | 5.3 | 2.8 | 2.9 |
| 0.2% Polyvinylpyrrolidone, MW=360K | No | >120 | - | - |
| 0.2% Polyvinylpyrrolidone, MW=360K | Yes | 1.6 | 1.9 | 1.9 |

| | | | | |
|---|---|---|---|---|
| ¹Southern Clay Products, Inc. ²Sasol North America, Inc. ³Aldrich, cat# 52,237-6. (The material is labeled by the supplier as "very low MW".) ⁴Acusol 480N, Rohm & Haas | | | | |

### TEST METHODS

Unless otherwise stated, all tests are performed under standard laboratory conditions (50% humidity and at 73°F (23°C)).

### Contact Angle

Dynamic contact angles are measured using the FTA200 Dynamic Contact Angle Analyzer, made by First Ten Angstroms, USA. A single drop of test solution is dispensed onto the sample substrate. A digital video recording is made while the drop spreads out across the surface of the substrate and the FTA200 software measures the contact angle of the liquid with the substrate as a function of time.

### Liquid Strike-Through Test

The liquid strike through time is measured using Lister-type strike-through equipment, manufactured by Lenzing AG, Austria. Test procedure is based on standardized EDANA (European Disposables And Nonwovens Association) method 150.3-96, with the test sample placed on an absorbent pad comprised of ten plies of filter paper (Ahlstrom Grade 632 obtained from Empirical Manufacturing Co., Inc. of 7616 Reinhold Drive, Cincinnati, OH 45237, USA, or equivalent). In a typical experiment, three consecutive 5ml gushes of test liquid (0.9% saline solution) are applied to a nonwoven sample at one minute intervals and the respective strike-through times are recorded without changing the absorbent pad.

## Claims

1. A method of rendering a material hydrophilic or increasing the hydrophilicity of a material, said method comprising the steps of:
(a) providing a material comprised of at least some hydrophobic or borderline hydrophilic components, wherein said material comprises a fabric material, and wherein said fabric material comprises a nonwoven material;
(b) applying a high energy surface treatment to said material to form a treated material; and
(c) applying a plurality of charged particles and at least one hydrophilic polymeric material to said treated material, said hydrophilic polymeric material comprising at least one of the following : a hydrophilic polymeric material having discrete charges; or a hydrophilic polymeric material with a strong dipole moment, wherein the particles have a size from 5 µm to less than 10 µm.

2. The method according to Claim 1 wherein the high energy surface treatment applied in step (b) comprises a treatment selected from the group consisting of: corona discharge treatment; plasma treatment; UV radiation; ion beam treatment; electron beam treatment; and laser treatment.

3. The method according to Claims 1 or 2 wherein steps (b) and (c) occur sequentially.

4. The method according to any one of Claims 1 to 3 wherein steps (b) and (c) occur simultaneously.

5. A method according to any one of Claims 1 to 4 wherein after step (c), the surface of the treated material becomes hydrophilic and has an advancing contact angle with water of less than 90°.

## Patentansprüche

1. Verfahren zum Hydrophilisieren eines Materials oder zum Erhöhen der Hydrophilie eines Materials, wobei das Verfahren die folgenden Schritte umfasst:
(a) Bereitstellen eines Materials, das aus wenigstens einigen hydrophoben oder grenzwertig hydrophilen Bestandteilen besteht, wobei das Material ein Stoffmaterial umfasst, und wobei das Stoffmaterial ein Vliesmaterial umfasst;
(b) Anwenden einer hochenergetischen Oberflächenbehandlung auf das Material, um ein behandeltes Material zu bilden; und
(c) Auftragen einer Vielzahl von geladenen Teilchen und mindestens eines hydrophilen Polymermaterials auf das behandelte Material, wobei das hydrophile Polymermaterial mindestens eines des Folgenden umfasst: ein hydrophiles Polymermaterial mit diskreten Ladungen, oder ein hydrophiles Polymermaterial mit einem starken Dipolmoment, wobei die Teilchen eine Größe von 5 µm bis weniger als 10 µm aufweisen.

2. Verfahren nach Anspruch 1, wobei die in Schritt (b) angewendete hochenergetische Oberflächenbehandlung eine Behandlung umfasst, die ausgewählt ist aus der Gruppe bestehend aus: Koronaentladungsbehandlung, Plasmabehandlung, UV-Bestrahlung, lonenstrahlbehandlung, Elektronenstrahlbehandlung und Laserbehandlung.

3. Verfahren nach den Ansprüchen 1 oder 2, wobei die Schritte (b) und (c) sequenziell erfolgen.

4. Verfahren nach einem der Ansprüche 1 bis 3, wobei die Schritte (b) und (c) gleichzeitig erfolgen.

5. Verfahren nach einem der Ansprüche 1 bis 4, wobei die Oberfläche des behandelten Materials nach Schritt (c) hydrophil wird und einen Fortschreitkontaktwinkel mit Wasser von weniger als 90° aufweist.

## Revendications

1. Procédé destiné à rendre un matériau hydrophile ou à augmenter l'hydrophilie d'un matériau, ledit procédé comprenant les étapes consistant à :
(a) fournir un matériau constitué d'au moins certains composants hydrophobes ou à peine hydrophiles, dans lequel ledit matériau comprend un matériau de tissu, et dans lequel ledit matériau de tissu comprend un matériau non tissé ;
(b) appliquer un traitement de surface à haute énergie audit matériau pour former un matériau traité ; et
(c) appliquer une pluralité de particules chargées et au moins un matériau polymère hydrophile audit matériau traité, ledit matériau polymère hydrophile comprenant au moins un des matériaux suivants : un matériau polymère hydrophile possédant des charges discrètes ; ou un matériau polymère hydrophile doté d'un fort moment dipolaire, dans lequel les particules ont une taille allant de 5 µm jusqu'à moins de 10 µm.

2. Procédé selon la revendication 1, dans lequel le traitement de surface à haute énergie appliqué dans l'étape (b) comprend un traitement choisi dans le groupe constitué d' : un traitement de décharge par effet de couronne ; un traitement au plasma ; un rayonnement UV ; un traitement par faisceau ionique ; un traitement par faisceau d'électrons ; et un traitement laser.

3. Procédé selon les revendications 1 ou 2, dans lequel les étapes (b) et (c) se produisent séquentiellement.

4. Procédé selon l'une quelconque des revendications 1 à 3, dans lequel les étapes (b) et (c) se produisent simultanément.

5. Procédé selon l'une quelconque des revendications 1 à 4, dans lequel, après l'étape (c), la surface du matériau traité devient hydrophile et présente un angle de contact d'avancement avec l'eau inférieur à 90°.
